(19) Europäisches Patentamt
European Patent Office
Office européen des brevets

(11) Veröffentlichungsnummer: **0 191 385**
**B1**

(12) # EUROPÄISCHE PATENTSCHRIFT

(45) Veröffentlichungstag der Patentschrift: **04.07.90**

(51) Int. Cl.[5]: **C07D 277/74**

(21) Anmeldenummer: **86101317.5**

(22) Anmeldetag: **01.02.86**

(54) **Verfahren zur Herstellung von Thiocyanatomethylthiobenzothiazolen.**

(30) Priorität: **13.02.85 DE 3504966**

(43) Veröffentlichungstag der Anmeldung:
**20.08.86 Patentblatt 86/34**

(45) Bekanntmachung des Hinweises auf die Patenterteilung:
**04.07.90 Patentblatt 90/27**

(84) Benannte Vertragsstaaten:
**DE FR GB IT**

(56) Entgegenhaltungen:
**DE-A-1 768 050**
**US-A-3 520 976**

**CHEMICAL ABSTRACTS, Band 104, Nr. 11, 17. März 1986, Seite 658, Zusammenfassung Nr. 88525z, Columbus, Ohio, US; & JP-A-60 132 971**

(73) Patentinhaber: **BAYER AG,**
**D-5090 Leverkusen 1 Bayerwerk(DE)**

(72) Erfinder: **Schade, Gerold, Dr., Hahnenweg 8,**
**D-5000 Köln 80(DE)**
Erfinder: **Wedemeyer, Karlfried, Dr., Bilharzstrasse 7,**
**D-5000 Köln 80(DE)**
Erfinder: **Diehl, Herbert, Dr., Walter-Flex-Strasse 17,**
**D-5090 Leverkusen 1(DE)**

EP 0 191 385 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft ein Verfahren zur Herstellung von Thiocyanatomethylthiobenzothiazolen durch Umsetzung von Halogenmethylthiobenzothiazolen mit Thiocyanaten.

Thiocyanatomethylthiobenzothiazole, die z.B. als Mikrobizide Verwendung finden, werden entsprechend dem US-Patent 3 520 976 hergestellt durch Umsetzung von Chlormethylthiobenzothiazolen mit Metallrhodanid in Gegenwart von organischen Lösungsmitteln.

Nachteilig bei diesem Verfahren ist die Gegenwart eines organischen Lösungsmittels, was bei der Übertragung in den großtechnischen Maßstab eine Reihe von Schwierigkeiten mit sich bringt.

So muß z.B. das organische Lösungsmittel zur Gewinnung des Produktes abgedampft werden, wobei Probleme dadurch auftreten können, daß die bei der Reaktion gebildeten Metallchloride an den wärmeübertragenden Flächen auskristallisieren. Die so entstehenden Verkrustungen behindern sehr stark den Wärmeübergang.

Nach dem Abdampfen des Lösungsmittels muß das Produkt zur Entfernung von Salzen aufwendigen Wasch- und Trocknungsoperationen unterworfen werden, wobei es wiederum mit einem Lösungsmittel verdünnt wird, das nachher entfernt werden muß.

Aus ökologischen Gründen ist die Wiedergewinnung und Reinigung der eingesetzten organischen Lösungmittel notwendig. Dies erfordert jedoch zusätzlichen technischen Aufwand, was ebenfalls zu Lasten der Wirtschaftlichkeit des Verfahrens geht.

Es wurde nun ein Verfahren zur Herstellung von Thiocyanatomethylthiobenzothiazolen der Formel

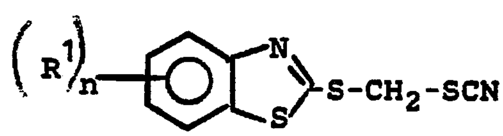 (I),

in der

$R^1$ für Wasserstoff, Halogen, eine Nitro-, Amino-, Hydroxy- oder eine Alkylgruppe steht und

n 1 oder 2 bedeutet,

durch Umsetzung von Halogenmethylthiobenzothiazolen der Formel

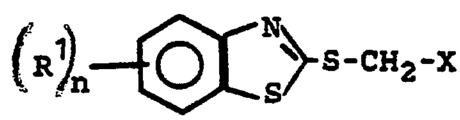 (II),

in der

$R^1$ und n die obengenannte Bedeutung haben und

X für Halogen steht,

mit Thiocyanaten der Formel

$$M^{\oplus}SCN^{\ominus} \qquad (III),$$

worin

M für ein Alkalimetall- oder für ein Ammoniumion steht,

bei erhöhter Temperatur gefunden, dadurch gekennzeichnet, daß man die Umsetzung in wäßriger Lösung in Gegenwart eines Phasentransferkatalysators vornimmt.

Als Halogene seien genannt: Fluor, Chlor, Brom und Jod, bevorzugt Fluor, Chlor und Brom, besonders bevorzugt Chlor;

als Alkylgruppen solche mit 1 bis 12 Kohlenstoffatomen, bevorzugt 1 bis 5 Kohlenstoffatomen, wie Methyl, Ethyl, n-Propyl, iso-Propyl, n-Butyl, iso-Butyl, tert.-Butyl, Pentyl, Hexyl, Heptyl, Octyl, Nonyl, Decyl, Undecyl und Dodecyl, bevorzugt Methyl, Ethyl, n-Propyl, iso-Propyl, n-Butyl, iso-Butyl, tert.-Butyl und Pentyl.

Besonders bevorzugt wird nach dem erfindungsgemäßen Verfahren das Thiocyanatomethylthiobenzothiazol der Formel (IV)

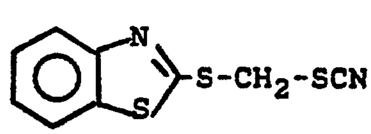 (IV)

hergestellt.

Zur Durchführung des erfindungsgemäßen Verfahrens werden die Halogenmethylthiobenzothiazole der Formel (II), insbesondere die entsprechenden 2-Chlormethylthiobenzothiazole, mit wäßriger Thiocyanatlösung bei erhöhter Temperatur in Gegenwart eines Phasentransferkatalysators umgesetzt. Als Phasentransferkatalysatoren können beispielsweise eingesetzt werden:

a) Quartäre Oniumsalze der allgemeinen Formel

$$AR^2R^3R^4R^5{}^{\oplus}X^{\ominus}$$

worin $R^2$ bis $R^5$ unabhängig voneinander für Alkyl, Aryl oder Aralkyl mit 1 bis 20 Kohlenstoffatomen stehen, A Stickstoff oder Phosphor bedeutet und X für Halogen, wie Fluor, Chlor, Brom oder Jod, bevorzugt Chlor oder Brom, Hydrogensulfat oder Thiocyanat stet. Beispielsweise seien folgende quartäre Oniumsalze genannt: Triethylbenzylammoniumchlorid, Tetrabutylammoniumbromid, -fluorid und -hydrogensulfat, Tricaprylylmethylammoniumchlorid, Hexadecyltrimethylammoniumbromid, Benzyldimethyldodecylammoniumjodid, Tetrabutylphosphoniumchlorid und Hexadecyltriphenylphosphoniumbromid.

Für den Fall, daß A in obiger Formel für Stickstoff steht, können auch mehrere der Reste $R^2$ bis $R^5$ zusammen einen Ring mit 4 bis 6 C-Atomen bilden. Dies ist z.B. beim Cetylpyridiniumchlorid der Fall.

b) Kronenether, wie 15-Krone-5, 18-Krone-6 und Dibenzo-18-Krone-6.

c) Polyether der allgemeinen Formel

$$R^6\text{-O-}(CH_2CH_2O)_x\text{-}R^7$$

worin $R^6$ und $R^7$ unabhängig voneinander für Wasserstoff, Alkyl oder gegebenfalls substituiertes Aryl mit 1 bis 20, bevorzugt 1 bis 12 Kohlenstoffatomen stehen und x eine ganze Zahl von 4 bis 40 bedeutet.

Beispielsweise seien genannt: Polyethylenglykol, Polyethylenglykolmonoethylether, Polyethylenglykoldimethylether, ethoxylierter Dodecylalkohol, ethoxyliertes Phenol und/oder ethoxyliertes Isononylphenol. Die hier genannten Polyether sind oftmals Gemische von Homologen, in denen x einen gewissen Bereich überstreicht.

Das vorliegende erfindungsgemäße Verfahren beschränkt sich nicht nur auf die obengenannten möglichen Phasentransferkatalysatoren, die nur eine Auswahl darstellen (vgl. weitere Literatur zu Phasentransferkatalysatoren, z.B. Dehmlow und Dehmlow, Phase Transfer Catalysis, Weinheim 1983).

Grundsätzlich sind für das erfindungsgemäße Verfahren alle Katalysatoren geeignet, die Thiocyanationen in ausreichender Konzentration in die organische Phase zu überführen vermögen.

Im allgemeinen werden die Phasentransferkatalysatoren beim erfindungsgemäßen Verfahren in Mengen von etwa 0,001 bis 0,1, bevorzugt 0,002 bis 0,01 Mol pro Mol eingesetztes Halogenmethylthiobenzothiazol eingesetzt. Sie können sowohl einzeln als auch im Gemisch untereinander eingesetzt werden.

Als Thiocyanate können die Alkalimetall- und/oder Ammoniumthiocyanate, bevorzugt das Natriumthiocyanat, verwendet werden. Ihre Konzentration in der wäßrigen Lösung ist nicht kritisch und kann im Bereich zwischen 20 Gew.-% und Sättigung liegen; bevorzugt liegt die Konzentration des Thiocyanats in der wäßrigen Lösung bei 30 bis 60 Gew.-%.

Nach dem erfindungsgemäßen Verfahren werden die Thiocyanate der Formel (III) in mindestens stöchiometrischen Mengen, bezogen auf die einzusetzenden Halogenmethylthiobenzothiazole eingesetzt. Ein Überschuß von bis zu 50 Gew.-% an Thiocyanat kann oftmals zweckmäßig sein.

Üblicherweise setzt man pro Mol Halogenmethylthiobenzothiazol der Formel (II) etwa 1,0 bis 1,2 Mole an Thiocyanat der Formel (III) in das erfindungsgemäße Verfahren ein.

Das erfindungsgemäße Verfahren wird im allgemeinen bei Temperaturen von etwa 70 bis 110°C, bevorzugt 75 bis 90°C durchgeführt. Arbeitet man im geschlossenen Gefäß, so kann die Reaktion auch unter Überdruck bei Temperaturen bis zu 120°C durchgeführt werden. Bei diesen Temperaturen ist beispielsweise das 2-Chlormethylthiobenzothialzol flüssig, so daß eine intensive Durchmischung des heterogenen Ansatzes möglich ist.

Die Dauer der Reaktion hängt vom Typ und von der Menge des eingesetzten Katalysators und der Reaktionstemperatur ab und beträgt im allgemeinen mehrere Stunden. Das Ende der Reaktion wird zweckmäßigerweise auf analytischem Wege, z.B. durch Gas- oder Dünnschichtchromatographie, festgestellt.

Ohne den Zusatz von Phasentransferkatalysatoren werden keine technisch brauchbaren Reaktionsgeschwindigkeiten erzielt.

Nach beendeter Reaktion erfolgt die Aufarbeitung in einfacher Weise beispielsweise durch Abtrennen des flüssigen Thicyanatomethylthiobenzothiazols von der wäßrigen Kochsalzlösung.

Obwohl die Beschleunigung von Substitutionsreaktionen in heterogenen Reaktionsgemischen durch den Zusatz von Phasentransferkatalysatoren eine an sich bekannte Methode auch für die Herstellung von organischen Thiocyanaten darstellt (vgl. DE-OS 1 768 050), war es dennoch nicht zu erwarten, daß auch beispielsweise Thiocyanatomethylthiobenzothiazol auf diesem Wege in glatter Reaktion hergestellt werden kann. Aus Liebigs Annalen Chem. 563, 54 (1949) ist nämlich bekannt, daß Chlormethylsulfide leicht hydrolysieren. So heißt es im zweiten Absatz auf Seite 57 der genannten Literaturstelle: "Sehr leicht zerfallen die α-halogenierten Thioether bei Berührung mit Wasser". So erfolgt die Hydrolyse nach folgendem Formelschema

$$2\ R\text{–}S\text{–}CH_2Cl + H_2O \rightarrow R\text{–}S\text{–}CH_2\text{–}S\text{–}R + H_2CO + 2\ HCl.$$

Aufgrund der bekannten Hydrolyseempfindlichkeit der α-halogenierten Thioether war es unwahrscheinlich, daß eine Phasentransferreaktion mit wäßriger Thiocyanatlösung beispielsweise Thiocyanatomethylthiobenzothiazol in guter Ausbeute und Reinheit liefern würde. Dies gilt insbesondere in Anbetracht der Reaktionsbedingungen, die für eine technisch brauchbare Umsetzungsgeschwindigkeit tatsächlich notwendig sind. Eine hohe Reinheit des gebildeten Produkts war jedoch unabdingbar, da Thiocyanatomethylthiobenzothiazol aufgrund seiner speziellen Eigenschaften nachträglich kaum gereinigt werden kann (vgl. DE-OS 1 670 221).

Um so überraschender war es deshalb, daß nach dem erfindungsgemäßen Verfahren die Thiocyanatomethylthiobenzothiazole in sehr guter Ausbeute und mit hohen Reinheiten erhalten wurden.

Das erfindungsgemäße Verfahren soll durch die folgenden Beispiele erläutert werden.

Beispiel 1

204,5 g 96 % Chlormethylthiobenzothiazol werden in 200 ml Wasser suspendiert und 90 g Natriumrhodanid sowie 2,3 g Benzyltriethylammoniumchlorid zugesetzt. Das Gemisch wird 10 h bei 80°C gerührt. Nach Abkühlen wird das ölig abgeschiedene Produkt abgetrennt. Ausbeute 226 g, Gehalt 82 %, Umsatz 97 %.

Beispiel 2

Arbeitet man wie bei Beispiel 1, jedoch bei 90°C, so erreicht man nach 7 h vollständigen Umsatz.

Beispiel 3

Arbeitet man wie bei Beispiel 1, jedoch mit ca. 1 Mol-% Tetrabutylammoniumbromid als Katalysator, so erreicht man nach 16 h vollständigen Umsatz.

Beispiel 4

Arbeitet man wie bei Beispiel 1, jedoch mit ca. 1 Mol-% 18-Krone-6 als Katalysator, so erreicht man nach 12 h 94 % Umsatz.

Beispiel 5

Arbeitet man wie bei Beispiel 4, jedoch mit ca. 5 Mol-% (bezogen auf die Struktureinheit $(CH_2CH_2O)_6$) Polyethylenglykol (mittlere Molmasse 1550) als Katalysator, so erreicht man nach 12 h 96 % Umsatz.

Beispiel 6

Arbeitet man wie bei Beispiel 5, jedoch mit der gleichen Gewichtsmenge 4-Isononylphenol-10-glykolether (NP 10) als Katalysator, so erreicht man nach 9,5 h 90 % Umsatz.

Beispiel 7

Arbeitet man wie bei Beispiel 1, jedoch im geschlossenen Gefäß bei 110°C, so erreicht man nach 5 h vollständigen Umsatz.

Beispiel 8 (Vergleichsbeispiel)

Arbeitet man wie bei Beispiel 1, jedoch ohne Katalysator so erreicht man nach 10 h nur 41 % Umsatz, nach 20 h nur 57 % und nach 30 h nur 68 % Umsatz.

**Patentansprüche**

1. Verfahren zur Herstellung von Thiocyanatomethylthiobenzothiazolen der Formel

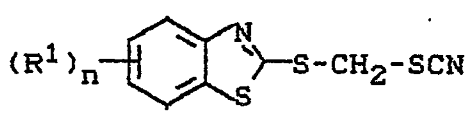 ,

in der
$R^1$ für Wasserstoff, Halogen, eine Nitro-, Amino-, Hydroxyl- oder eine Alkylgruppe steht und
n 1 oder 2 bedeutet,
durch Umsetzung von Halogenmethylthiobenzothiazolen der Formel

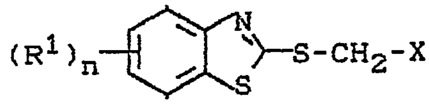

,

in der
$R^1$ und n die obengenannte Bedeutung haben und
X für Halogen steht,
mit Thiocyanaten der Formel

$$M^+SCN^-,$$

worin
M für ein Alkalimetall oder für Ammonium steht,
bei erhöhter Temperatur, dadurch gekennzeichnet, daß man die Umsetzung in wäßriger Lösung in Gegenwart eines Phasentransferkatalysators vornimmt.

2. Verfahren nach Anspruch 1, dadurch gekennzeichnet, daß man die Umsetzung bei Temperaturen von 70 bis 110°C durchführt.

3. Verfahren nach Ansprüchen 1 und 2, dadurch gekennzeichnet, daß man als Phasentransferkatalysatoren quartäre Oniumsalze, Kronenether und/oder Polyether einsetzt.

4. Verfahren nach Ansprüchen 1 bis 3, dadurch gekennzeichnet, daß man die Phasentransferkatalysatoren in Mengen von 0,001 bis 0,1 Mol pro Mol Halogenmethylthiobenzothiazol einsetzt.

**Claims**

1. Process for the preparation of thiocyanatomethylthiobenzothiazoles of the formula

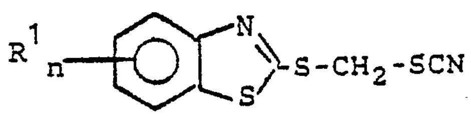

in which
$R^1$ represents hydrogen, halogen, a nitro, amino, hydroxyl or an alkyl group and
n denotes 1 or 2,
by reacting halomethylthiobenzothiazoles of the formula

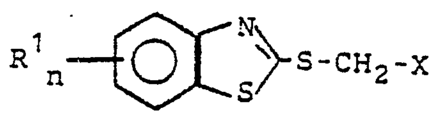

in which
$R^1$ and n have the abovementioned meaning and
X represents halogen,
with thiocyanates of the formula

$$M^+SCN^-$$

wherein
M represents an alkali metal or ammonium, at elevated temperatutre, characterized in that the reaction is performed in aqueous solution in the presence of a phase transfer catalyst.

2. Process according to Claim 1, characterized in that the reaction is carried out at temperatures of 70 to 110°C.

3. Process according to Claims 1 and 2, characterized in that quaternary onium salts, crown ethers and/or polyethers are used as phase transfer catalysts.

4. Process according to Claims 1 to 3, characterized in that the phase transfer catalysts are used in quantities of 0.001 to 0.1 mol per mol of halomethylthiobenzothiazole.

**Revendications**

1. Procédé pour la fabrication de thiocyanatométhylthiobenzothiazoles de formule

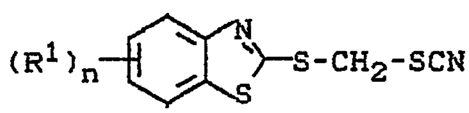

,

dans laquelle
$R^1$ représente l'hydrogène ou un halogène ou un groupe nitro, amino, hydroxyle ou alkyle et
n est égal à 1 ou 2,
par réaction d'halogénométhylthiobenzothiazoles de formule

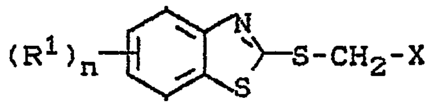

,

dans laquelle
$R^1$ et n ont la signification ci-dessus et
X représente un halogène,
avec des thiocyanates de formule

$$M^+SCN^-$$

dans laquelle
M représente un ion de métal alcalin ou d'ammonium, à température élevée, caractérisé en ce que l'on effectue la réaction en solution aqueuse en présence d'un catalyseur de transfert de phase.

2. Procédé selon la revendication 1, caractérisé en ce que l'on met en œuvre la réaction à des températures de 70 à 110°C.

3. Procédé selon les revendications 1 et 2, caractérisé en ce que l'on utilise comme catalyseurs de transfert de phase des sels onium quaternaires, des éthers en couronne et/ou des polyéthers.

4. Procédé selon les revendications 1 à 3, caractérisé en ce que l'on utilise les catalyseurs de transfert de phase en quantités de 0,001 à 0,1 mol par mol d'halogénométhylthiobenzothiazole.